# EUROPEAN PATENT SPECIFICATION

(11) **EP 0 982 037 B1**
(45) Date of publication and mention of the grant of the patent: **06.06.2007**
(21) Application number: 99909244.8
(22) Date of filing: 19.03.1999
(51) Int. Cl.: A61K 38/17, A61K 38/18, A61P 43/00

(54) **USE OF TCF-II FOR PREVENTING OR TREATING SEPSIS**
VERWENDUNG VON TCF-II ZUR BEHANDLUNG ODER VORBEUGUNG VON SEPSIS
UTILISATION DE TCF-II POUR LE TRAITEMENT OU LA PREVENTION DE LA SEPTICEMIE

(30) Priority: 19.03.1998 JP 7091498
(43) Date of publication of application: 01.03.2000
(73) Proprietor: DAIICHI PHARMACEUTICAL CO., LTD., Chuo-ku, Tokyo 103-8234 (JP)
(72) Inventor: TANI, Tohru, Kusatsu-shi Shiga-ken 525-0055 (JP); KONDO, Hiroyuki, Otsu-shi Shiga-ken 520-2143 (JP)
(74) Representative: Wakerley, Helen Rachael
(86) International application number: PCT/JP1999/001373
(87) International publication number: WO 1999/047155

(56) References cited:
- EP-A- 0 588 477
- WO-A1-90/10651
- DATABASE MEDLINE [Online] US NATIONAL LIBRARY OF MEDICINE (NLM), BETHESDA, MD, US; February 1996 (1996-02), ZENG W ET AL: "[Prevention of endotoxic shock in rats with hepatic stimulating substance]" XP002292879 Database accession no. NLM9275627 & ZHONGHUA NEI KE ZA ZHI [CHINESE JOURNAL OF INTERNAL MEDICINE]. FEB 1996, vol. 35, no. 2, February 1996 (1996-02), pages 99-102, ISSN: 0578-1426
- HERNANDEZ J ET AL: "CHARACTERIZATION OF THE EFFECTS OF HUMAN PLACENTAL HGF ON RAT HEPATOCYTES" JOURNAL OF CELLULAR PHYSIOLOGY, vol. 150, no. 1, 1992, pages 116-121, XP008034125 ISSN: 0021-9541
- KONDO H ET AL: "EFFECTS OF DELETION-TYPE HUMAN HEPATOCYTE GROWTH FACTOR ON MURINE SEPTIC MODEL" JOURNAL OF SURGICAL RESEARCH, ACADEMIC PRESS INC., SAN DIEGO, CA, US, vol. 85, no. 1, July 1999 (1999-07), pages 88-95, XP001104926 ISSN: 0022-4804

## Description

### Technical field

The present invention relates to the use of Tumor Cytotoxic Factor-II (TCF-II) as an effective ingredient in the manufacture of a medicament for preventing and/or treating sepsis. By the present invention, a therapeutic agent for preventing and/or treating sepsis caused by more than one factor selected from the group consisting of infectious disease, burn, surgery, cancer, acquired immunodeficiency syndrome (AIDS), radiotherapy, chemotherapy, long term total parenteral nutrition (TPN) is provided and useful as a medicine.

### Prior art

Sepsis is generally severe systemic infectious disease which developes by continuous or intermittent invasion of bacteria or products secreted by them into blood from bacterial infectious focus present somewhere in tissue or organ of human body. Sepsis is induced by chemotherapy using corticosteroid or antitumor agent, etc., radiotherapy like cobalt radiation, or treatment or operation such as self-retaining catheter, blood dialysis, organ transplantation, heart surgery etc. in a patient with malignant tumor, leukemia, malignant lymphoma, AIDS, collagen disease, renal insufficiency, hepatic disease, cerebrovascular disease or diabetes or in a host with decreased resistance such as humoral immunodeficiency or cellular immunodeficiency of an aged person or an immature infant. Recently, though various kinds of antibiotic have been developed and clinically used for the treatment of sepsis, various kinds of antibiotic-resistant bacterium, such as penicillin- or methicillin-resistant Staphylococcus aureus, emerged, so that mortality rate of patients with sepsis is still about 25 % and many patients die due to sepsis every year.

As bacteria which induce sepsis, in addition to the afore- mentioned antibiotic-resistant bacteria, enterostreptococcus, gram-negative bacillus such as Escherichia coli, Pseudomonas aeruginosa, Klebsiella and Proteus, etc. are exemplified. Among these, a patient with sepsis due to gram-negative bacillus suffers from hyperthermia, chill trepidation, tachycardia, slight depression of blood pressure, depression of peripheral resistance, hyperkinemia, hyperventilation, respiratory alkalosis, hyperlactacidemia and sometimes heart failure at early stage, followed by depression of blood pressure and cardiac output and falling into severe conditions accompanying with metabolic acidosis, consiousness affection, acroagnosis, hypoglycemia and sometimes resulting in death. Therefore, treatment of sepsis is considered to be an important object.

Bacteria are mainly thought to invade into living body by two different kinds of route. One is exogenous case such as intravenous self-retaining catheter or puncture needle used on infusion or supplemental infusion, and the other is endogenous case such as invasion of bacteria widely distributed in human living body such as intestine etc.

Transfer of bacteria or products secreted by them from intestine is called as bacterial translocation or endotoxin translocation and is noticed as a trigger of inducing sepsis. Such a translocation of bacteria and/or products secreted by them from intestine is observed when a patient is severely damaged by burn, major surgery, etc, and when a patient after surgery or a patient with consciousness affection receives long term TPN treatment and becomes serious problems.

For the treatment of sepsis comprising these bacterial translocation, antibiotic treatment is chosen first and recognized to have a certain level of effects. However, antibiotic treatment is thought to have its limit due to its side-effects, appearance of antibiotic-resistant bacteria and its little effects on shock symptom. In addition, supplement of nutrition by infusion or treatment by γ-globulin is carried out but these are aimed for recovery of host immunity and remain as auxiliary treatment for antibiotic treatment. Thus, there is no effective treatment for sepsis.

### Disclosure of the invention

The present inventors have eagerly studied to find out a therapeutic agent for sepsis and found that TCF-II known as tumor cytotoxic factor had an excellent effect for preventing and treating sepsis. Accordingly, an object of the present invention is to provide a therapeutic agent for preventing and/or treating sepsis caused by more than one factor selected from the group consisting of burn, surgery, cancer, AIDS, radiotherapy, chemotherapy and long term TPN, comprising TCF-II as an effective ingredient.

The present invention provides the use of TCF-II as an effective ingredient in the manufacture of a medicament for preventing and/or treating sepsis. The therapeutic agent is useful for preventing and/or treating sepsis caused by more than one factor selected from the group consisting of burn, surgery, cancer, AIDS, radiotherapy, chemotherapy and long term TPN.

TCF-II is an effective ingredient of the present invention, a known protein derived from human fibroblast and has the following properties:
1) Molecular weight (SDS electrophoresis)
   under non-reducing conditions; 78,000 ± 2,000 or 74, 000 ± 2, 000
   under reducing conditions; 52,000 ± 2,000 (common band A)
   30, 000 ± 2, 000 (band B)
   26,000 ± 2,000 (band C)
2) Isoelectric point; 7.4-8.6

The above TCF-II can be obtained by a method of condensing culture medium of human fibroblast, adsorbing the condensate on ionexchange resin and purifying the eluent by affinity column chromatography (WO90/10651) or by a genetically engineered manipulation (WO92/01053).

TCF-II as an effective ingredient of the present invention can be TCF-II derived from human fibroblast IMR-90 or TCF-II genetically engineered using microorganism or other cell lines based on the genetic sequence described in patent publication WO90/10651. TCF-II obtained by genetically engineered manipulation described in patent publication WO92/01053 can be also used. In such a case, TCF-II with different polysaccharide chain or without polysaccharide chain due to the difference of host cell or microorganism can be used but TCF-II with polysaccharide chain is preferable. TCF-II obtained by these methods can be condensed and purified by usual isolation and purification method. For example, precipitation using organic solvent, salting-out, gel filtration, affinity chromatography using monoclonal antibody and electrophoresis can be exemplified. Purification by affinity chromatography using monoclonal antibody can use monoclonal antibody described in Japanese Published unexamined patent application No. 97 (1993). Obtained purified TCF-II can be lyophilized or kept freezed. Other substances showing the same activity as TCF-II can be used as the same therapeutic agent. For example, hepatocyte growth factor (HGF; Japanese Published unexamined patent application No. 22526/1988) which is different from TCF-II protein in five amino acids or Purified Scatter Factor (SF; Gherardi and Stocker Nature, 346, 228 (1990)) can be exemplified.

The therapeutic agent for preventing and/or treating sepsis can be administered intravenously, intramuscularly or subcutaneously as injections. These preparations can be produced according to a known pharmaceutical preparation method and pH conditioners, buffers, stabilizers can be added thereto if necessary. Dose of the therapeutic agent for a patient depends on degree of symptom, health conditions, age and body weight of a objected patient. Though it is not especially restricted, 0.6-600 mg, preferably 6- 60 mg, of purified TCF-II can be administered once a day for one adult patient.

### Brief description of the drawings

Figure 1 shows the effects of TCF-II on model animals of cecum-punctured sepsis in example 1.
   ● represents vehicle-administered group and
   ■ represents TCF-II-administered group
Figure 2 shows the effects of TCF-II on LPS-induced bacterial translocation in example 2.
   □ represents vehicle-administered group and
   ■ represents TCF-II-administered group

### Best mode for carrying out the invention

The present invention will be described more specifically by the following examples but these are merely exemplified and the scope of the present invention is not restricted by these examples.

### [Preparative example 1]

### Purification of TCF-II

According to a method described in WO90/10651 and a method of Higashio et. al. (Higashio, K. et. al., B. B. R. C., vol. 170, pp397-404 (1990)), cell was cultured and purified TCF-II was obtained. That is, 3 x 10⁶ human fibroblast IMR-90 (ATCC CCL186) cells were placed in a roller bottle containing 100 ml DMEM medium including 5 % calf fetal serum and cultured by rotating it at the rate of 0.5-2 rpm for 7 days. When the total number of cell reached 1 x 10⁷ cells were deprived from the wall by trypsin digestion and collected at the bottom of bottle. And 100g of ceramic with the size of 5 - 9 mesh (Toshiba Ceramic) was sterilized and put therein, which was cultured for 24 hours. After then, 500 ml of the above culture medium was added thereto and the culture was continued. The total volume of culture medium was recovered every 7-10 days and fresh medium was supplemented. Production was kept for 2 months like this and 4 liters of culture medium was recovered per a roller bottle. Specific activity of TCF-II in culture medium obtained as above was 32 µ g/ml. Culture medium (750 L) was concentrated by ultrafiltration using membrane filter (MW 6,000 cut; Amicon) and purified by 4-steps chromatography, that is, CM- Sephadex C-50 (Pharmacia), Con-A Sepharose (Pharmacia), Mono S column (Pharmacia), Heparin-Sepharose (Pharmacia) to yield purified TCF-II.

### [Preparative example 2]

### Production of recombinant TCF-II

According to a method described in WO92/01053, cell transformed with TCF-II gene was cultured and purified TCF-II was obtained. That is, transformed Namalwa cell was cultured and 20 l of culture medium was obtained. This culture medium was treated by CM-Sephadex C-50 chromatography, Con-A Sepharose CL-6B chromatography and finally HPLC equipped with a Mono S column to yield about 11 mg of recombinant TCF-II.

### [Preparative example 3]

### Production of pharmaceutical preparation of TCF-II

An example of producing injections of TCF-II obtained preparative examples 1 and 2 was shown below/

| | | |
|---|---|---|
| (1) | TCF-II | 20 µg |
| | human serum albumin | 100 mg |

The above composition was dissolved in citric acid buffer solution with pH 6.03 so that the total volume would be 20 ml.
Then, it was divided into vials containing 2 ml each after sterilization and sealed after lyophilization.

| | | |
|---|---|---|
| (2) | TCF-II | 40 µg |
| | Tween 80 | 1 mg |
| | Human serum albumin | 100 mg |

The above composition was dissolved in physiological saline solution for injections so that the total volume would be 20 ml.
Then it was divided into vials containing 2 ml each after sterilization and sealed after lyophilization.

| | | |
|---|---|---|
| (3) | TCF-II | 20 µg |
| | Tween 80 | 2 mg |
| | Sorbitol | 4 g |

The above composition was dissolved in citric acid buffer solution with pH 6.03 so that the total volume would be 20 ml.
Then it was divided into vials containing 2 ml each after sterilization and sealed after lyophilization.

| | | |
|---|---|---|
| (4) | TCF-II | 40 *µ*g |
| | Tween 80 | 1 mg |
| | Glycine | 2 g |

The above composition was dissolved in physiological saline solution for injections so that the total volume would be 20 ml.
Then it was divided into vials containing 2 ml each after sterilization and sealed after lyophilization.

| | | |
|---|---|---|
| (5) | TCF-II | 40 *µ*g |
| | Tween 80 | 1 mg |
| | Sorbitol | 2 g |
| | Glycine | 1 g |

The above composition was dissolved in physiological saline solution for injections so that the total volume would be 20 ml.
Then it was divided into vials containing 2 ml each after sterilization and sealed after lyophilization.

| | | |
|---|---|---|
| (6) | TCF-II | 20 *µ*g |
| | Sorbitol | 4 g |
| | Human serum albumin | 50 mg |

The above composition was dissolved in citric acid buffer solution with pH 6.03 so that the total volume would be 20 ml.
Then it was divided into vials containing 2 ml each after sterilization and sealed after lyophilization.

| | | |
|---|---|---|
| (7) | TCF-II | 40 *µ*g |
| | Glycine | 2 g |
| | Human serum albumin | 50 mg |

The above composition was dissolved in physiological saline solution for injections so that the total volume would be 20 ml.

Then it was divided into vials containing 2 ml each after sterilization and sealed after lyophilization.

| | | |
|---|---|---|
| (8) | TCF-II | 40 µg |
| | Human serum albumin | 50 mg |

The above composition was dissolved in citric acid buffer solution with pH 6.03 so that the total volume would be 20 ml.
Then it was divided into vials containing 2 ml each after sterilization and sealed after lyophilization.

### [Example 1]

### Effects of TCF-II in septic rats made by puncturing cecum

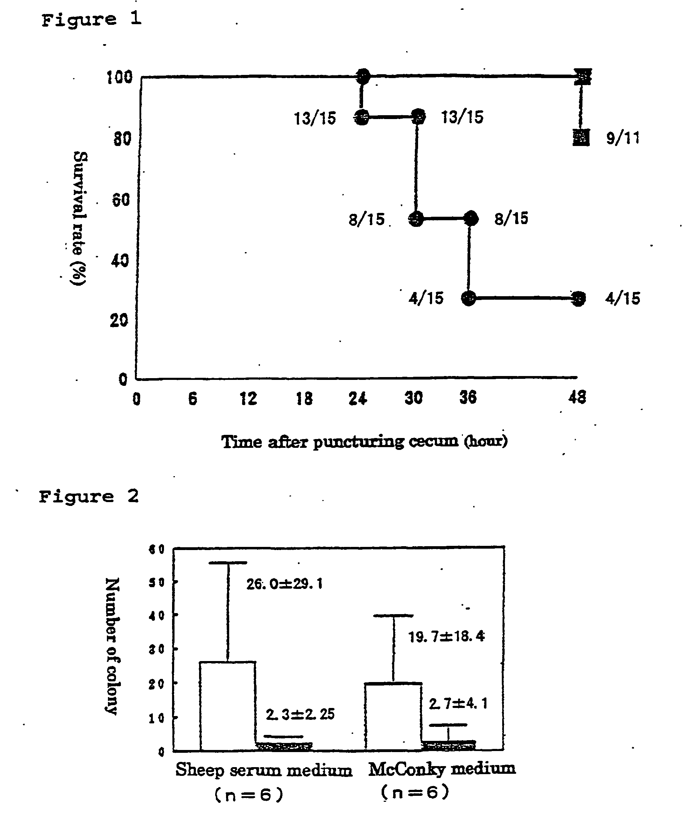

Septic model animals were made by puncturing cecum once with 22G injection needle using 7 week old male SD rats (J. Surgical Research 29, 189-201 (1980)). At 48 hours before making septic model, animals were divided into two groups consisting of a control group which would be administered with vehicle only (vehicle-administered group) (n=15) and a TCF-II-administered group (n=11). They were intravenously administered with vehicle or TCF-II (1000 µ g/kg) 5 times every 12 hours. The result of the change of survival rate of animals until 2 days after making septic model was shown in figure 1. As the results, the survial rate on day 2 of TCF-II administered group was significantly higher than that of vehicle administered group, which demonstrated that TCF-II administration significantly increased in the survival rate of septic rats. That is, it was confirmed that TCF-II improved the survival rate of individuals with sepsis.

### [Example 2]

### Effects of TCF-II administration on LPS-induced bacterial translocation

LPS was intravenously administered in 7 weeks old male ICR mice (5mg/kg). At 30 hours before LPS administration, animals were divided into 2 groups consisting of a control group which would be administered with vehicle only (vehicle-administered group) (n=6) and TCF-II-administered group (n=6). They were intravenously administered with vehicle or TCF-II (200 µ g/kg) 3 times every 12 hours. The result of the number of bacteria detected in mesenteric lymph node at 24 hours after LPS administration was shown in figure 2. The detected number of bacteria in the TCF-II-administered group was significantly lower than that of the control group, which demonstrated that TCF-II administration significantly suppressed LPS-induced translocation of bacteria from intestine.
That is, it was confirmed that TCF-II significantly improved the survival rate against bacterial translocation which can be a trigger of inducing sepsis.

### Industrial utility

By the present invention, TCF-II as an effective ingredient in the manufacture of a medicament for preventing or treating sepsis is provided. The therapeutic agent is useful for preventing and/or treating sepsis caused by more than one factor selected from the group consisting of infectious disease, burn, surgery, cancer, AIDS, radiotherapy, chemotherapy, long term TPN

## Claims

1. Use of Tumor Cytotoxic Factor-II (TCF-II) as an effective ingredient in the manufacture of a medicament for preventing and/or treating sepsis.

## Patentansprüche

1. Verwendung von tumorzytotoxischem Faktor II (TCF-II) als Wirkstoff bei der Herstellung eines Medikaments zur Verhütung und/oder Behandlung von Sepsis.

## Revendications

1. Utilisation du Facteur-II de cytotoxicité tumorale (TCF-II) en tant qu'ingrédient efficace dans la fabrication d'un médicament pour la prévention et/ou le traitement du sepsis.
